# FASCICULE DE BREVET EUROPEEN

(11) **EP 2 092 842 B1**
(45) Date de publication et mention de la délivrance du brevet: **21.12.2016**
(21) Numéro de dépôt: 09152947.9
(22) Date de dépôt: 16.02.2009
(51) Int. Cl.: A45D 26/00, A61B 18/20

(54) **Dispositif d'épilation utilisant une radiation électromagnetique pulsée**
Enthaarungsvorrichtung, die pulsierende elektromagnetische Strahlung benutzt
Hair-removal device using pulsed electromagnetic radiation

(30) Priorité: 22.02.2008 FR 0851170
(43) Date de publication de la demande: 26.08.2009
(73) Titulaire: Brottier, Yves Vincent, 78113 Adainville (FR)
(72) Inventeur: Brottier, Yves Vincent, 78113 Adainville (FR)
(74) Mandataire: Cabinet Plasseraud

(56) Documents cités:
- EP-A- 0 788 814
- EP-A- 0 885 629
- WO-A-2004/047921
- US-B1- 6 174 325
- US-B1- 6 228 074

## Description

La présente invention concerne les appareils d'épilation utilisés par applications cutanées locales et émettant des impulsions de rayonnement visible et/ou infrarouge (V/IR), ces impulsions étant générées par une source d'énergie électromagnétique incohérente telle qu'une lampe flash - généralement au xénon - dans laquelle se décharge de l'énergie électrique préalablement stockée dans un ou plusieurs condensateurs.

Plus particulièrement, l'invention concerne un dispositif destiné à une épilation, comportant un boîtier, une lampe flash, un coupleur, un filtre intercalé entre la lampe flash et le coupleur, un premier agencement de fixation pour fixer le filtre dans le boîtier, un deuxième agencement de fixation pour fixer la lampe flash dans le boîtier, le coupleur permettant de diriger de l'énergie électromagnétique issue de la lampe flash sur la surface d'une peau.

Ces appareils produisent des impulsions dont la durée est généralement comprise entre 5 et 80 ms, ce qui est exceptionnellement long pour un flash, et présente, pour cette raison, des problèmes spécifiques de dissipation de chaleur.

Dans ce type d'appareil, les lampes flash de forte puissance (dédiées à la photothérapie) pour dispenser une lumière intense pulsée ont un système de refroidissement à eau. Typiquement, le tube au xénon est baigné dans une cavité fermée dans laquelle circule le liquide de refroidissement. Ce liquide est mis en circulation par une pompe. Il passe dans un radiateur pour permettre une diffusion thermique. Le radiateur est associé à un puissant ventilateur de dimensions importantes.

Un des inconvénients des systèmes de refroidissement à eau est la nécessité de garantir un circuit parfaitement étanche, notamment au niveau de la lampe. Il en résulte systématiquement une élévation de l'encombrement et du poids de l'appareil. De surcroît, il apparaît régulièrement une pollution du liquide de refroidissement qui entraîne une pollution de la lampe (parois). Un vieillissement prématuré de la lampe oblige à effectuer un changement de la lampe, et occasionne une indisponibilité de l'appareil plus fréquente.

Un système de refroidissement qui comprend un circuit de fluide autour de la lampe flash est utilisé dans le dispositif d'épilation décrit dans le document WO 2004/047921 A1. Le dispositif présente un boîtier qui permet de loger la lampe flash et présente une ouverture à travers laquelle l'énergie électromagnétique de la lampe flash peut être transmise. A l'intérieur du boîtier, le fluide servant au refroidissement est mis en circulation, soit par des moyens de pompage, soit par un ventilateur. L'utilisation d'un ventilateur implique cependant une perte de compacité de l'épilateur.

Il existe donc un besoin pour des appareils compacts qui ne présentent pas les inconvénients majeurs liés à un circuit de refroidissement à eau.

La présente invention a donc pour but de fournir un dispositif d'épilation qui soit équipé d'un système peu encombrant pour diffuser de manière efficace l'échauffement généré par l'utilisation de la lampe flash.

A cet effet, le boîtier du dispositif d'épilation comprend :
- une cavité adjacente à la lampe flash ;
- au moins un orifice d'aération ; et
- au moins un organe mobile dans le boîtier, déplaçable entre une première position, dite d'aération, dans laquelle ladite cavité communique avec ledit orifice d'aération et une deuxième position, dite de fermeture, pour laquelle ladite cavité est fermée de façon à limiter des échappements de lumière en dehors de l'ouverture.

Grâce à ces dispositions, il est permis d'obtenir un dispositif d'épilation par flash dont l'échauffement est efficacement limité et qui présente une économie de coût du fait de la disparition des tuyauteries, de la pompe et du radiateur, sans oublier les différents joints. De plus l'encombrement est réduit. Un simple ventilateur local de format réduit ou même la diffusion par la convection naturelle peut suffire à ventiler la surface du filtre entre deux flashes.

De plus, l'applicateur qui intègre la lampe peut être conçu comme un élément autonome, avec ou sans cordon de liaison à une base qui n'inclut pas de circuit de refroidissement par exemple. Il en résulte une plus grande facilité de manipulation, permettant à une personne seule d'effectuer l'épilation sur n'importe quelle surface de sa propre peau. A titre d'exemple non limitatif, le boîtier peut présenter un organe rapprochant, lorsque la lampe flash est fixée, l'interface ou coupleur d'extrémité du dispositif vers la lampe flash ou vice versa, par exemple par un simple mouvement de poussée.

Selon une autre particularité, le boîtier présente :
- une première portion de boîtier présentant l'ouverture ; et
- une deuxième portion de boîtier autorisant un déplacement en translation entre la première portion de boîtier et la deuxième portion de boîtier, la lampe flash étant solidaire de la deuxième portion de boîtier, l'organe mobile étant formé de l'une des première et deuxième portions.

Ainsi, le dispositif selon l'invention reste simple de conception avec une portion mobile par rapport à l'autre maintenue fixe lorsque le boîtier est refermé.

Selon une autre particularité, la première portion de boîtier est une tête et la deuxième portion de boîtier forme un manche préhensible d'une main qui permet de rapprocher la lampe flash vers l'extrémitc libre de la tête lorsque la tête est en appui sur une surface extérieure au dispositif. Ainsi, le boîtier est aisé à manipuler et l'actionnement peut être effectué de façon intuitive juste après avoir disposé la tête en appui via le coupleur sur la surface de peau à traiter.

Selon une autre particularité, le dispositif comprend à l'intérieur du boîtier :
- un coupleur permettant de diriger l'énergie électromagnétique issue de la lampe flash sur la surface d'une peau ;
- un filtre intercalé entre la lampe flash et le coupleur ;
- un premier agencement de fixation pour fixer le filtre dans le boîtier ; et
- un deuxième agencement de fixation pour fixer la lampe flash dans le boîtier ;
l'organe mobile déplaçant l'un au moins des premier et deuxième agencements de fixation, la lampe flash étant disposée sensiblement en vis-à-vis et à distance du filtre pour ladite première position, la lampe flash et le filtre étant étroitement rapprochés pour ladite position de fermeture.

Ainsi, la surface du filtre peut être refroidie à chaque période intermédiaire entre deux flashes.

Selon une autre particularité, le premier agencement de fixation possède un degré de liberté en translation entre le coupleur et la lampe flash, le premier agencement de fixation étant maintenu à distance du coupleur par au moins un organe de sollicitation élastique placé en périphérie du coupleur. Ainsi, la force de rappel élastique exercée sur le premier agencement de fixation permet de disposer par défaut le filtre à distance du coupleur. On comprend que l'actionnement de la liaison ou mécanisme d'entraînement permet simultanément un rapprochement de la lampe vers le filtre d'une part, et du filtre vers le coupleur d'autre part.

Selon une autre particularité, le premier agencement de fixation comprend un support de forme tubulaire qui comprend sur le côté opposé au coupleur, des languettes élastiques de clipsage retenant entre elles le filtre, le boîtier comportant une portion tubulaire entourant le support et permettant de guider le support. Ainsi, du fait de la présence du support, le filtre peut être déplacé en translation sans risque d'être altéré mécaniquement.

Selon une autre particularité, le deuxième agencement de fixation forme avec la lampe flash un consommable extractible du boîtier et comprend un caisson pour loger la lampe flash, ledit caisson présentant :
- une face d'extrémité présentant une fenêtre pour laisser échapper des rayons de la lampe flash ;
- des premiers éléments de fixation définissant des extrémités opposées à ladite face d'extrémité;
- des seconds éléments de fixation disposés en périphérie de la fenêtre ;
les premiers éléments de fixation étant configurés pour s'engager avec au moins un organe allongé suivant une première direction, tandis que les seconds éléments de fixation sont configurés pour s'engager avec au moins un organe allongé suivant une deuxième direction perpendiculaire à la première direction.

Grace à ces dispositions, le consommable s'intègre parfaitement à l'intérieur du boîtier du dispositif d'épilation en générant un minimum d'encombrement et contribue de manière effective à la cinématique de rapprochement relatif entre la lampe flash et le filtre.

Selon une autre particularité, le boîtier présente intérieurement une surface de guidage de flux d'air parallèlement au filtre et à partir de laquelle s'étend, selon une direction générale perpendiculaire au filtre, ledit organe allongé. La surface de guidage permet de guider le flux d'air entre le filtre et la lampe dans la position active du dispositif (par exemple 2-3 mm).

Selon une autre particularité, ledit organe allongé présente un épaulement et se termine en une portion de tige d'extrémité, le caisson comprenant au moins un évidement avec un fond et un ressort prévu dans l'évidement, l'évidement permettant de recevoir au moins en partie ledit organe allongé, le ressort présentant une première.extrémité s'appuyant sur le fond et une deuxième extrémité s'appuyant sur l'épaulement de l'organe allongé, ledit ressort étant comprimé pour ladite position de fermeture dans laquelle la lampe flash et le filtre sont étroitement rapprochés. Grâce à ces dispositions, le caisson logeant la lampe reprend automatiquement sa position de repos, en retrait par rapport au filtre.

Selon une autre particularité, les premiers éléments de fixation comprennent des éléments conducteurs d'alimentation métalliques pour alimenter la lampe flash. Ainsi, la lampe flash peut être alimentée par un générateur intégré dans le boîtier, par exemple dans la partie formant manche. Ceci permet d'optimiser l'ergonomie des composants à l'intérieur du boîtier. L'appareil constitué du boîtier, de la lampe flash, du filtre et du coupleur forme un ensemble autonome. On comprend que la présence d'une liaison électriquement conductrice dans des éléments de fixation participant à l'entraînement permet à l'utilisateur d'effectuer des rechanges du consommable sans avoir à débrancher/brancher des connecteurs supplémentaires à l'intérieur du boîtier.

Selon une autre particularité, le deuxième agencement de fixation comprend un caisson permettant de loger la lampe flash et présentant des éléments conducteurs d'alimentation métalliques s'étendant à l'opposé du coupleur, la deuxième portion de boîtier mobile présentant au moins une pièce métallique sensiblement cylindrique sur laquelle se fixent les éléments conducteurs d'alimentation.

Ainsi, le contact électrique vers la lampe peut être effectué via des cylindres constitués d'une pleine masse d'un matériau conducteur, par exemple le cuivre (ce qui permet d'optimiser l'efficacité de l'alimentation de la lampe flash).

Selon une autre particularité, le caisson présente une fenêtre qui laisse s'échapper une pluralité de rayons de la lampe flash suivant une orientation générale déterminée, la première portion de boîtier présentant au moins une pièce de guidage du caisson allongée suivant un axe parallèle à ladite orientation générale déterminée. Ainsi, le mouvement de la lampe flash est sécurisé, seule une translation rectiligne étant permise.

Selon une autre particularité, le dispositif comprend un générateur auquel est reliée la lampe flash, un capteur apte à délivrer une information représentative de l'occupation par ledit organe mobile de ladite position de fermeture, et un dispositif de contrôle associé au générateur configuré pour recevoir au moins une information du capteur et permettre l'émission d'un seul flash lumineux lorsque ladite position de fermeture a été détectée par le capteur. Ainsi, la multiplication de flash lumineux sur une même surface de traitement est empêchée dans un bref laps de temps.

Par ailleurs, l'invention a également pour objet un consommable pour un dispositif conforme à l'invention, caractérisé en ce qu'il comprend :
- une lampe flash ;
- un caisson pour loger la lampe flash, présentant une face d'extrémité avec une fenêtre pour laisser échapper des rayons de la lampe flash ;
- du côté de la face d'extrémité et en périphérie de la fenêtre, au moins un évidement dans lequel est placé un ressort qui présente une extrémité mobile entre une position comprimée en retrait permettant à là cavité du dispositif d'être fermée de façon à limiter des échappements de lumière hormis vers l'ouverture et une position avancée pour laquelle la cavité communique avec l'orifice d'aération du dispositif ; et
- au moins un élément de fixation du caisson au boîtier, présentant une surface de butée qui est orientée suivant un sens opposé par rapport au sens de poussée du ressort

Ainsi, le consommable muni de ses ressorts permet de conserver dans une.position de repos (entre deux flashes) la communication de la cavité avec le ou les orifices d'aération du boîtier. Des passages sont par exemple aménagés dans le caisson, de part et d'autre de la fenêtre, pour assurer la communication d'air vers la cavité.

D'autres caractéristiques et avantages de l'invention apparaîtront au cours de la description suivante de plusieurs modes de réalisation, donnés à titre d'exemples non limitatifs, en regard des dessins joints dans lesquels :
- les figures 1A et 1B représentent chacune une vue de face et respectivement de profil, de la tête d'un dispositif d'épilation selon l'invention ;
- la figure 2 est une vue en perspective d'un consommable incluant la lampe flash dans un mode de réalisation de l'invention ;

- la figure 3 représente une vue en perspective d'un dispositif d'épilation selon l'invention pour la position au repos ;
- la figure 4A. est une vue selon le plan de coupe A-A de la figure 1A de la tête du dispositif d'épilation pour la position au repos du dispositif ;
- la figure 4B est une vue selon le plan de coupe B-B de la figure 1B de la tête du dispositif d'épilation pour la position au repos du dispositif ;
- la figure 5A représente une vue selon le plan de coupe A-A de la figure 1A de la tête du dispositif d'épilation pour la position active du dispositif ;
- la figure 5B représente une vue selon le plan de coupe B-B de la figure 1B de la tête du dispositif d'épilation pour la position active du dispositif ;
- la figure 6 est une vue en perspective illustrant la position du filtre dans la tête du dispositif d'épilation pour la position actionnée du dispositif ;
- la figure 7 représente une coque supérieure du manche d'un dispositif d'épilation selon l'invention ;
- la figure 8 représente une vue éclatée d'un dispositif d'épilation conforme à l'invention.

Sur les différentes figures, les mêmes références désignent des éléments identiques ou similaires. Dans ce qui suit, on entend par lampe flash 10 toute source d'énergie électromagnétique incohérente adaptée pour un traitement d'épilation. Plus généralement, la phraséologie et les différentes terminologies employées servent uniquement à des fins de description et ne doivent pas être interprétées de façon limitative.

Selon la présente invention, il est permis de réaliser une épilation en exposant une zone pileuse à une intense énergie électromagnétique sous la forme de flashes lumineux. Cette énergie chauffe les poils et affecte en particuliers les follicules sans engendrer de dommage sur une peau saine. Un gel optiquement transparent d'un type connu en soi est préférentiellement appliqué avant le traitement. Le gel sert à supprimer la lame d'air comprise entre l'extrémité du coupleur et la surface de l'épiderme. Le gel contribue donc à l'efficacité de la transmission optique.

Le dispositif d'épilation 11 comprend une lampe flash 10, par exemple et de manière nullement limitative au xénon, permettant de couvrir une zone relativement étendue, par exemple entre 4 et 50 cm². Cela permet de réduire le temps de traitement. La forme du faisceau peut être de forme régulière rectangulaire ou de forme symétrique analogue avec une surface utile comprise entre 6 et 44 cm².

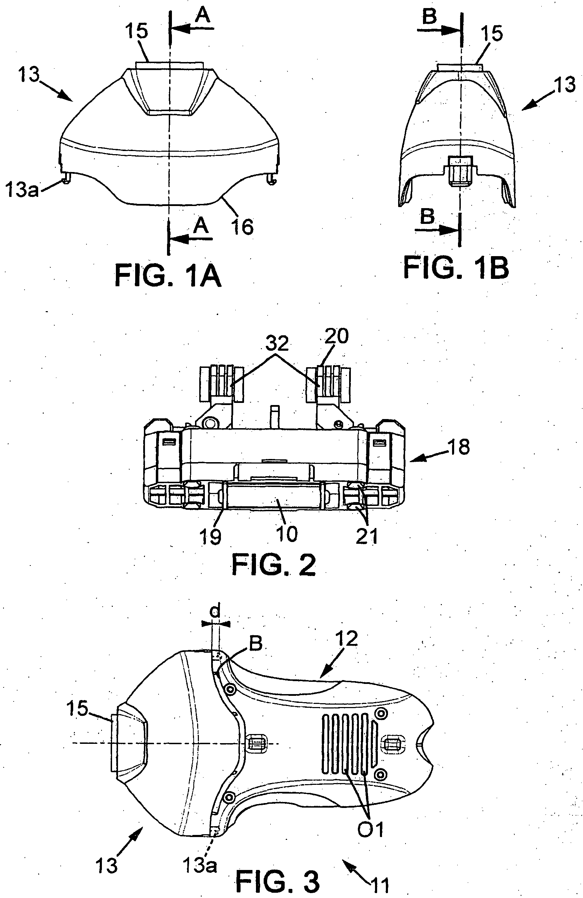

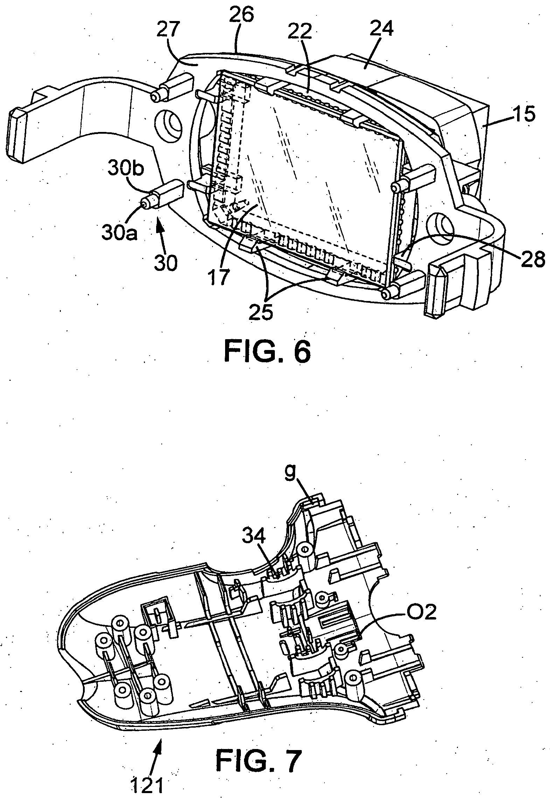

En référence maintenant aux figures 1A, 1B, 2 et 3, la lampe flash 10 est logé à l'intérieur d'un boîtier qui peut se décomposer en deux portions de boîtier (12, 13) dont l'une forme un manche 12 et l'autre une tête 13. L'assemblage entre ces deux portions de boîtier (12, 13) peut être réalisé par encliquetage via au moins deux languettes périphériques 13a de la tête 13 coopérant chacune avec une gorge g (figure 7), rainure ou cavité analogue du manche 12 qui laisse une liberté de mouvement selon un axe longitudinal traversant le manche 12 et la tête 13. Naturellement, d'autres modes appropriés d'assemblage peuvent être utilisés.

A titre d'exemple comme illustré à la figure 3, la tête 13 présente une ouverture 14 d'extrémité traversée par un coupleur 15 qui permet de diriger l'énergie électromagnétique issue de la lampe flash 10 sur la surface d'une peau lors d'un traitement d'épilation effectué par le dispositif 11. Autrement dit, le coupleur 15 définit un conduit optique entre la lampe flash 10 et la surface de peau à traiter, préférentiellement enduite d'un gel. Le coupleur 15 peut être au moins à base de quartz ou d'autre matériau solide transparent. Le coupleur 15 peut être constitué d'une ou plusieurs pièces; il peut s'agir d'une simple vitre.

Une source d'alimentation (non représentée) peut être placée à l'intérieur du manche 12 et connectée à la lampe flash 10. La source d'alimentation peut être autonome et rechargée via un connecteur prévu dans le manche 12 pour se brancher à une base de gestion du dispositif d'épilation 11 ou à un réseau urbain. Le boîtier illustré dans les figures est préhensible et portable d'une seule main. Le dispositif d'épilation 11 formé par le boîtier et son contenu peut ainsi délivrer une pluralité de flashes de façon indépendante et autonome.

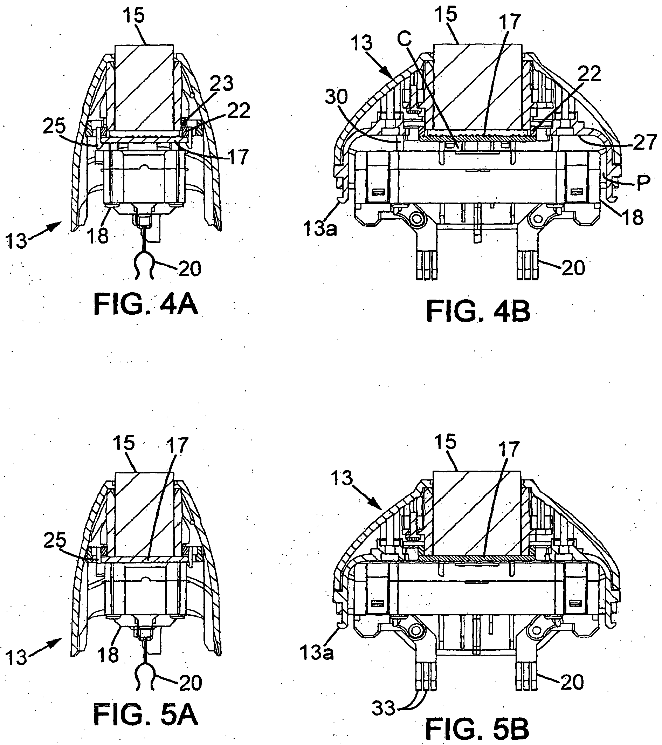

En référence à la figure 3, le coupleur 15 optique placé dans la tête 13 dépasse en saillie par rapport à l'ouverture 14 d'extrémité de la tête 13. Le volume intérieur de la portion de boîtier fermant la tête 13 permet de loger l'essentiel du coupleur 15. De plus, la forme évasée de la tête 13 dans le mode de réalisation représenté permet de faire circuler l'air latéralement de la lampe flash 10 disposée à l'arrière au coupleur 15, ainsi que dans un espace ou cavité C (figure 4B) définie entre la lampe flash 10 et le coupleur 15.

Dans le mode de réalisation représenté, le manche 12 peut être actionné dans son ensemble par l'utilisateur selon un mouvement de poussée en translation rectiligne en direction de l'extrémité libre de la tête 13 maintenue contre une surface à traiter pour déclencher un flash. Lors du fonctionnement, la surface à traiter bloque la tête 13, ce qui permet par un mouvement de poussée de déplacer le manche 12. Ce mouvement de translation étant relatif, on peut aussi indifféremment considérer que le manche 12 est maintenu fixe et que la tête 13 s'enfonce dans le manche 12, ou encore que ces deux portions de boîtier se déplacent toutes deux l'une vers l'autre. L'étendue du déplacement peut correspondre sensiblement à la distance d entre la bordure 16 du pourtour de la tête 13 et une bordure B extérieure adjacente du manche 12. La bordure 16 à l'arrière de la tête 13 définit une ouverture suffisamment grande pour permettre l'insertion de l'extrémité avant du manche 12 dans le volume intérieur de la tête 13.

Le déplacement du manche 12 est permis par l'espace laissé entre la lampe flash 10 et le coupleur 15, à l'intérieur de la tête 13. Un système de rappel élastique maintient par défaut l'écartement intérieur de la portion de boîtier formant le manche 12 par rapport à la portion de boîtier formant la tête 13, de l'air pouvant ainsi aisément circuler dans cet espace entre deux flashes.

La liaison d'entraînement associée au manche 12 va être à présent décrite en référence aux figures 2, 4A, 4B, 5A, 5B et 6.

De façon connue en soi, la lampe flash 10 correspond à une source électromagnétique logée dans un caisson 18, l'ensemble pouvant former un consommable (10, 18) extractible du boîtier. Comme montre à la figure 2, le caisson 18 présente une face d'extrémité avec une fenêtre 19 pour laisser échapper les rayons de la lampe flash 10. Le caisson 18 comprend également d'une part des premiers éléments de fixation 20 disposés à l'opposé de la fenêtre, et d'autre part des seconds éléments dé fixation 21 disposés en périphérie de la fenêtre 19. Les premiers éléments de fixation 20 assurent la liaison mécanique du consommable (10, 18) avec le manche 12, tandis que les seconds éléments de fixation 21 contribuent à caler le consommable (10, 18) dans une position déterminée par rapport à la tête 13 du dispositif d'épilation 11. La fixation permise par ces éléments respectifs (20, 21) est de type amovible et le consommable (10, 18) peut être retiré manuellement, sans l'aide d'outil.

La liaison mécanique du consommable (10, 18) avec Le manche 12 permet de pousser la lampe flash 10 vers lé coupleur 15 lorsque l'utilisateur actionne le manche 12. Naturellement, l'actionnement permettant de déplacer la lampe flash 10 peut aussi être initié de façon différente, en fonction de la structure de l'organe mobile ou du mécanisme d'entraînement choisi pour permettre ce déplacement.

Comme indiqué à la figure 6, un filtre 17 au moins est prévu dans le boîtier pour filtrer le faisceau issu de la lampe flash 10. Ce filtre 17 peut être disposé entre la lampe flash 10 et le coupleur 15. Ce filtre 17, ou dans un mode de réalisation alternatif plusieurs filtres, sont utilisés pour contrôler le spectre généré par la lampe flash 10. Un filtre 17 d'absorption peut ainsi être utilisé. Dans certaines conditions d'utilisation, le filtre 17, pourrait aussi être supprimé.

En référence aux figures 4A, 4B, 5A et 5B, le dispositif d'épilation 11 selon l'invention possède une position désactivée ou position de repos (figures 4A et 4B) et une position activée (figures 5A et 5B). Dans la position de repos, le circuit de ventilation ou d'aération ventile le filtre 17 via la ou les cavité(s) adjacente(s) C au filtre 17. La cavité C communique avec l'extérieur dans cette position, par exemple par l'intermédiaire de passages latéraux P. Dans la position activée, le caisson 18 logeant la lampe flash 10. est déplacé pour rapprocher étroitement la lampe flash 10 du filtre 17. Après ce rapprochement relatif entre le caisson 18 et la tête 13, la cavité C adjacente à la lampe flash 10 est isolée du reste du boîtier et forme une enceinte étroite qui limite les échappements latéraux de lumière et peut ainsi favoriser la transmission optique vers l'ouverture 14. Du côté opposé à cette ouverture 14, la lampe flash 10 est entourée d'un réflecteur agencé dans le caisson 18. La cavité définie par le réflecteur est prolongée par la cavité C. On comprend que le fait de quitter la position activée amène un apport d'air devant la fenêtre 19, l'air arrivant via des passages traversant le caisson 18 et/ou des passages latéraux. L'air peut traverser le caisson 18 dans dés espaces aménagés, essentiellement au niveau des électrodes externes de la lampe flash 10 (passages sur l'extérieur du réflecteur comme cela est visible sur la figure 2 par exemple). Une ventilation de la cavité C est ainsi obtenue. On comprend que l'aménagement réservé dans le caisson 18 peut servir de canalisation d'air provenant par exemple d'un ventilateur embarqué dans le dispositif 11 et à destination de la cavité C, et permet aussi avantageusement de refroidir les électrodes.

Dans le mode de réalisation représenté, le filtre 17 est sensiblement aplati et disposé dans le boîtier dans sa position intermédiaire à l'aide d'un agencement de fixation solidaire de la tête 13. Comme montré notamment à la figure 6, cet agencement de fixation comprend un support 22 de forme tubulaire, par exemple en forme de cadre, avec des dimensions comparables à celles du filtre 17. Ce support 22 possède un degré dé liberté en translation entre le coupleur 15 et la lampe flash 10 installée dans le boîtier, suivant un axe perpendiculaire à un plan défini par le filtre 17.

Comme montré à la figure 4A qui correspond à une position en retrait du consommable (10, 18) logeant la lampe flash 10, un ou plusieurs organes de sollicitation élastique 23 tels que des ressorts sont placés longitudinalement, par exemple dans une disposition périphérique autour du coupleur 15, afin de maintenir le support 22 et le filtre 17 à distance du coupleur 15. Ces organes de sollicitation élastique 23 prennent par exemple appui sur un cadre ou une couronne intérieure 24 formée autour du coupleur 15, au niveau de l'extrémité de la tête 13.

Pour la position activée du dispositif d'épilation 11 qui est montrée dans les figues 5A et 5B, ces organes de sollicitation élastique 23 peuvent être comprimés par au moins une face d'appui du support 22 du filtre 17. Dans cette position activée, le consommable (10, 18) et le filtre 17 sont tous deux rapprochés du coupleur 15. Un contact est par exemple établi entre le filtre 17 et le coupleur 15 dans cette position. Le contact de poussée du support 22 du filtre 17 par le caisson 18 s'effectue à distance du filtre 17, par l'intermédiaire de languettes 25 de clipsage retenant entre elles le filtre 17 et s'étendant vers l'arrière de la tête 13. Les languettes 25 de clipsage qui présentent des propriétés élastiques sont au moins au nombre de trois, et en particulier au nombre de quatre dans le mode de réalisation représenté à la figure 6 (position activée).

Dans sa position avancée (position activée du dispositif), comme illustré à la figure 5A, le caisson 18 vient en appui sur l'extrémité libre des languettes 25 de clipsage et enfonce ainsi l'ensemble du support 22 du filtre 17 vers l'avant de la tête 13. La position enfoncée du support 12 du filtre 17 est représenté à la figure 6. La fin de course pour cet enfoncement du support 22 peut être prescrite par la jonction entre la bordure 16 de la tête 13 et la bordure B du manche. Ainsi, le filtre 17 maintenu sur son support 22 peut être positionné de façon précise contre le coupleur sans être déformé, ce qui contribue au maintien de l'intégrité du filtre 17. Dans le mode de réalisation représenté à la figure 3, les bordures (13, B) ont une forme complémentaire. Dans l'exemple illustré aux figures 5A et 5B, cette position enfoncée correspond à une mise en contact du filtre 17 contre le coupleur 15.

En référence à la figure 6, la portion de boîtier formant la tête 13 peut comporter une portion tubulaire 26 qui entoure le support 22 et permet de guider ce dernier. La portion tubulaire 26 présente par exemple une surface intérieure avec des fentes. Des éléments en saillie 28 ou projections radiales du support 22 peuvent coulisser dans ces fentes de guidage.

La portion tubulaire 26 définit également une surface 27 orientée vers l'arrière de la tête 13 et permettant de guider un flux d'air parallèlement au filtre 17, comme illustré à la figure 4B dans la position désactivée du dispositif 11. Le fait que le filtre 17 soit déplaçable permet de définir dans le boîtier, pour la position désactivée, des espaces de part et d'autre du filtre 17 afin de permettre une refroidissement de ce dernier. Le mouvement de translation ou autre mouvement approprié permet d'accroître l'espacement entre le filtre et la lampe flash lors du retour à la position désactivée, de sorte que l'air pénétrant dans le volume intermédiaire ainsi créé peut refroidir efficacement par convection la surface du filtre 17. Le fait de pouvoir ventiler le filtre 17 sur ses deux faces permet un refroidissement efficace lorsque l'énergie délivrée est élevée. De plus, on empêche ainsi au maximum le filtre 17 de transmettre son énergie thermique au conduit optique défini par le coupleur 15. Un refroidissement sur une seule face peut également être satisfaisant pour des applications avec un moindre niveau d'énergie. La séparation ou mise à distance du filtre 17 par rapport aux éléments qui accumulent de la chaleur (tube à xénon et réflecteur) coupe avantageusement les possibilités de transmission de chaleur, notamment vers le coupleur 15. La finesse du filtre 17 le rend aussi plus facile à refroidir.

Le boîtier peut incorporer un ou plusieurs ventilateurs (non représentés) de format réduit, par exemple au niveau du manche 2. En référence aux figures 4A et 4B, la cavité ou espace compris entre la face avant du caisson 18 et le filtre 17 peut être ventilé à l'intérieur du boîtier via des passages traversant et/ou contournant le caisson 18. De la même façon, l'espace formé entre le filtre 17 et le coupleur 15 va pouvoir être ventilé. Le débouché des passages à travers le caisson 18 peut être par exemple décalé latéralement vers l'extérieur par rapport aux extrémités du filtre 17. L'air sortant de ces passages peut ensuite, dans la position de repos, pleinement circuler dans la ou les cavité(s) C adjacente(s) au filtre 17. On comprend ainsi que le filtre 17 est efficacement refroidi entre deux flashes. On comprend que la mobilité vers la position activée du dispositif 11 permet de limiter les déperditions de lumière (amélioration du rendement énergétique). La mobilité vers la position de repos du dispositif 11 permet de réduire l'état de confinement autour de la lampe flash 10 qui limite les possibilités d'aération efficace de cette dernière.

Comme cela est visible sur les figures 4B, 5B et 6, le boîtier présente également un ou plusieurs organes allongés 30 qui s'étendent depuis la surface 27 de guidage vers l'intérieur du boîtier jusqu'à une extrémité libre 30a en forme de téton, selon une direction générale perpendiculaire au filtre 17 et suivant un sens opposé au sens de rayonnement. Ces organes allongés 30 pénètrent par leur extrémité libre 30a dans des évidements du caisson 18. Ces évidements de forme cylindrique forment les seconds éléments de fixation 21 du caisson 18. Le caisson 18 est ainsi rendu solidaire en rotation de la portion de boîtier formant la tête 13. Les organes allongés 30 laissent en revanche une liberté de mouvement en translation suivant un axe longitudinal du dispositif d'épilation 11. Le caisson 18 est ainsi guidé en translation par ces organes allongés 30 qui peuvent se présenter sous la forme de tiges.

En référence à la figure 6, l'organe allongé 30 présente un épaulement 30b et se termine en une portion de tige jusqu'à l'extrémité libre 30a. Le caisson 18 présente au moins un évidement avec un fond et un ressort (non représenté) prévu dans l'évidement, l'évidement permettant de recevoir au moins en partie ledit organe allongé 30, le ressort présentant une première extrémité s'appuyant sur le fond et une deuxième extrémité s'appuyant sur l'épaulement 30b, ledit ressort étant comprimé pour la deuxième position dans laquelle la lampe flash 10 et le filtre 17 sont étroitement rapprochés.

Dans le mode de réalisation représenté à la figure 5B correspondant à la position activée du dispositif d'épilation 11, chacun des organes allongés 30 pénètre en totalité dans l'évidement correspondant du caisson 18. Ce dernier vient alors s'appuyer par sa face d'extrémité avant sur la surface 27 de guidage. On comprend que dans la position activée, la cavité ou espace pour l'aération du filtre 17 est supprimé ou réduit et coupé des passages d'aération. Pour éviter à un utilisateur de déclencher plusieurs flashes dans cette position, il est par exemple prévu dans le boîtier un capteur qui délivre une information représentative de l'occupation de la position activée avec rapprochement entre la lampe flash 10 et le filtre 17. Un dispositif de contrôle associé au générateur qui alimente la lampe flash 10 est également prévu dans le boîtier pour recevoir une ou plusieurs informations de ce capteur et permettre l'émission d'un seul flash lumineux lorsque la position activée a été détectée par le capteur.

En pratique, après avoir choisi la zone à traiter, l'utilisateur doit exercer une pression sur le manche 12 pour obtenir la position activée, un flash étant alors déclenché. Ensuite, l'utilisateur relâche la pression et laisse le manche 12 revenir dans sa position par défaut de désactivation. Pendant le temps de relâchement de pression, le dispositif 11 peut être déplacé pour être appliqué sur la zone suivante à traiter.

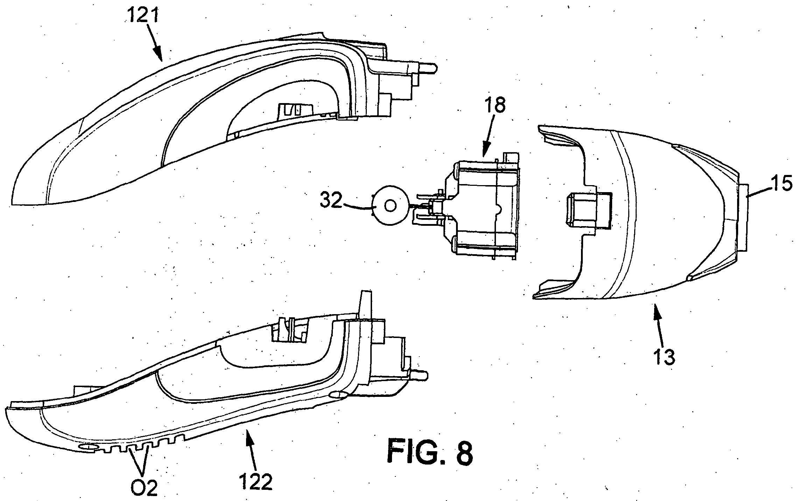

En référence à présent aux figures 3, 7 et 8, la portion de boîtier formant le manche 12 peut présenter des orifices O1 d'amenée d'air extérieur, tandis que des orifices 02 de refoulement d'air intérieur peuvent être prévus au niveau de la jonction entre le manche 12 et la tête 13. Deux coques (121, 122) complémentaires peuvent être assemblées pour former le manche 12, comme illustré à la figure 8. La coque inférieure 122 présente par exemple plusieurs orifices 02 répartis le long du manche 12. Le générateur d'alimentation électrique de la lampe flash 10 peut être logé à l'arrière du boîtier. Des cylindres 32 en cuivre, maintenus par des supports 34 concaves formés intégralement avec les coques (121, 122) du manche 12, sont en contact électrique direct avec une partie d'extrémité intérieure des connecteurs électriques du caisson 18. La masse importante de ces cylindres 32 apporte une inertie thermique, importante permettant de réduire l'échauffement au niveau des contacts électriques. Dans un mode de réalisation, la taille des électrodes externes du tube de la lampe flash 10 est importante pour limiter les échauffements thermiques. Ces électrodes externes s'étendent par exemple sur plus d'un centimètre. Pour accroître leur inertie thermique, elles peuvent présenter un diamètre important et une longueur relativement importante.

Le caisson 18 peut être équipé de lames contact 33 ressorts à base d'un alliage cuivre-béryllium CuBe₂. Ces lames de contact 33 forment aussi au moins une partie des premiers éléments de fixation 20 prévus pour fixer le caisson 18 à la portion de boîtier formant le manche 12. Ces lames de contact 33 s'étendent à l'opposé du coupleur 15 et sont espacées les une par rapport aux autres et agencées en forme de collier ouvert de clipsage, comme illustré aux figures 4A à 5B. Les lames de contact 33 peuvent ainsi se fixer sur des organes, tels que des cylindres, allongés transversalement suivant un axe perpendiculaire aux organes allongés 30 guidant le mouvement de translation du caisson 18 logeant la lampe flash 10. Dans le caisson 18, la connexion avec les électrodes externes de la lampe flash 10 peut se présenter sous une forme complètement similaire à celles des lames de contact 33.

Le matériau béryllium CuBe₂ présente des caractéristiques optimisées pour la conduction électrique, la conduction thermique et l'élasticité nécessaire au "clipsage"/"déclipsage" lors du changement du consommable (10, 18). Certaines parties en CuBe₂ et en cuivre peuvent être dorées à l'or fin pour faciliter les contacts électriques 300 V/300 A. cette partie de connexion électrique est par exemple ventilée par un ou plusieurs ventilateurs placés dans le manche 12.

L'assemblage du consommable (10, 18) dans le boîtier peut être réalisé de façon simple par l'utilisateur, par exemple lors du remplacement de la lampe flash 10. Le boîtier peut être d'abord séparé en deux par enlèvement de la tête 13. Le caisson 18 est ensuite couplé au manche 12 par les premiers éléments de fixation 20 (via les cylindres 32). Il s'agit d'un clipsage. Il reste ensuite à fermer le boîtier en venant clipser la tête 13 sur le manche 12 par les deux languettes 13a. Le dispositif est alors assemblé et opérationnel.

Un des avantages du dispositif est une meilleure gestion de l'énergie qui permet d'égaler les caractéristiques du meilleur matériel professionnel dans un encombrement totalement optimisé.

Il doit être évident pour les personnes versées dans l'art que la présente invention permet des modes de réalisation sous de nombreuses autres formes spécifiques sans l'éloigner du domaine d'application de l'invention comme revendiqué. En particulier, bien que la description soit relative à un appareil dont une partie du boîtier formant le manche 12 peut actionner l'entraînement par poussée, on comprend que tout autre mode d'actionnement peut être utilisé (rotation, mouvement hélicoïdal, traction, activation d'un bouton ou d'une gâchette, etc.) pour conduire à un résultat analogue. Ainsi, une structure de support du filtre 17 et/ou du coupleur 15 peut être rendue mobile relativement au reste du boîtier qui comprend la lampe flash 10. L'appui sur la surface de peau à traiter permet par exemple d'actionner le déplacement de cette structure mobile dans ce cas. Par ailleurs, le découplage entre deux flashes peut uniquement consister en une séparation du filtre 17 par rapport à la lampe flash 10 (découplage simple) au lieu de séparer le filtre 17 à la fois de la lampe flash 10 et du coupleur 15 (découplage double). Une ventilation de toute la surface du filtre 17 reste assurée de manière efficace avec un découplage simple. Alternativement ou de façon complémentaire, la fermeture de la cavité C peut être aussi déclenchée par utilisation d'un ou plusieurs organes mobiles déplaçant des parois d'obturation de passages de communication avec la cavité C ou tout espace similaire adjacent à la lampe flash 10.

## Revendications

1. Dispositif (11) destiné à une épilation, comportant un boîtier, une lampe flash (10) disposée à l'intérieur du boîtier, le boîtier présentant une ouverture (14) à travers laquelle de l'énergie électromagnétique issue de la lampe flash (10) peut être transmise vers la surface d'une peau, le boîtier comportant une cavité (C) adjacente à la lampe flash (10), **caractérisé en ce que** le boîtier comprend :
- au moins un orifice d'aération (01,02) ; et
- au moins un organe mobile, déplaçable entre une première position, dite d'aération, dans laquelle ladite cavité (C) communique avec ledit orifice d'aération (01,02) et une deuxième position, dite de fermeture, pour laquelle ladite cavité (C) est fermée de façon à limiter des échappements de lumière en dehors de l'ouverture (14).

2. Dispositif selon la revendication 1, dans lequel le boîtier présente :
- une première portion de boîtier qui comporte ladite ouverture (14) ; et
- une deuxième portion de boîtier autorisant un déplacement en translation entre la première portion de boîtier et la deuxième portion de boîtier, la lampe flash (10) étant solidaire de la deuxième portion de boîtier, l'organe mobile étant formé de l'une des dites première et deuxième portions.

3. Dispositif selon la revendication 2, dans lequel la première portion de boîtier est une tête (13) et la deuxième portion de boîtier forme un manche (12) préhensible d'une main, ledit organe mobile permettant de rapprocher la lampe flash (10) de l'extrémité libre de la tête (13) lorsque la tête (13) est en appui sur une surface extérieure au dispositif (11).

4. Dispositif selon une des revendications 1 à 3, comprenant à l'intérieur du boîtier :
- un coupleur (15) permettant de diriger l'énergie électromagnétique issue de la lampe flash (10) sur la surface d'une peau ;
- un filtre (17) intercalé entre la lampe flash (10) et le coupleur (15) ;
- un premier agencement de fixation (22, 25) pour fixer le filtre (17) dans le boîtier ; et
- un deuxième agencement de fixation pour fixer la lampe flash (10) dans le boîtier ;
ledit organe mobile déplaçant l'un au moins des premier et deuxième agencements de fixation, la lampe flash (10) étant disposée sensiblement en vis-à-vis et à distance du filtre (17) pour ladite position d'aération, la lampe flash (10) et le filtre (17) étant étroitement rapprochés pour ladite position de fermeture.

5. Dispositif selon la revendication 4, dans lequel le premier agencement de fixation (22, 25) possède un degré de liberté en translation entre le coupleur (15) et la lampe flash (10), le premier agencement de fixation (22, 25) étant maintenu à distance du coupleur (15) par au moins un organe de sollicitation élastique (23) placé en périphérie du coupleur (15).

6. Dispositif selon une des revendications 4 et 5, dans lequel le premier agencement de fixation (22, 25) comprend un support (22) de forme tubulaire qui comprend sur le côté opposé au coupleur (15), des languettes (25) élastiques de clipsage retenant entre elles ledit filtre (17), le boîtier comportant une portion tubulaire (26) entourant le support (22) et permettant de guider le support (22).

7. Dispositif selon une des revendications 4 à 6, dans lequel le deuxième agencement de fixation forme avec la lampe flash (10) un consommable (10, 18) extractible du boîtier et comprend un caisson (18) pour loger la lampe flash (10), ledit caisson (18) présentant :
- une face d'extrémité présentant une fenêtre (19) pour laisser échapper des rayons de la lampe flash (10) ;
- des premiers éléments de fixation (20) définissant des extrémités opposées à ladite face d'extrémité;
- des seconds éléments de fixation (21) disposés en périphérie de la fenêtre (19) ;
les premiers éléments de fixation (20) étant configurés pour s'engager avec au moins un organe allongé (30) suivant une première direction, tandis que les seconds éléments de fixation (21) sont configurés pour s'engager avec au moins un organe allongé (30) suivant une deuxième direction perpendiculaire à la première direction.

8. Dispositif selon la revendication 7, dans lequel le boîtier présente intérieurement une surface (27) de guidage d'un flux d'air parallèlement au filtre (17) et à partir de laquelle s'étend, selon une direction générale perpendiculaire au filtre (17), ledit organe allongé (30).

9. Dispositif selon une des revendications 7 et 8, dans lequel ledit organe allongé (30) présente un épaulement (30b) et se termine en une portion de tige d'extrémité, le caisson (18) comprenant au moins un évidement avec un fond et un ressort prévu dans l'évidement, l'évidement permettant de recevoir au moins en partie ledit organe allongé (30), le ressort présentant une première extrémité s'appuyant sur le fond et une deuxième extrémité s'appuyant sur l'épaulement (30b) de l'organe allongé(30), ledit ressort étant comprimé pour ladite position de fermeture dans laquelle la lampe flash (10) et le filtre (17) sont étroitement rapprochés.

10. Dispositif selon une des revendications 7 à 9, dans lequel les premiers éléments de fixation (20) comprennent des éléments conducteurs d'alimentation (33) métalliques pour alimenter la lampe flash (10).

11. Dispositif selon une des revendications 9 à 10, dans lequel le deuxième agencement de fixation comprend un caisson (18) permettant de loger la lampe flash (10) et présentant des éléments conducteurs d'alimentation (33) métalliques s'étendant à l'opposé du coupleur (15), la deuxième portion de boîtier mobile présentant au moins une pièce métallique sensiblement cylindrique (32) sur laquelle se fixent les éléments conducteurs d'alimentation (33).

12. Dispositif selon la revendication 11, dans lequel le caisson (18) présente une fenêtre (19) qui laisse s'échapper une pluralité de rayons de la lampe flash (10) suivant une orientation générale déterminée, la première portion de boîtier présentant au moins une pièce de guidage du caisson (18) allongée suivant un axe parallèle à ladite orientation générale déterminée.

13. Dispositif selon une des revendications 1 à 12, comprenant un générateur auquel est reliée la lampe flash (10), un capteur apte à délivrer une information représentative de l'occupation par ledit organe mobile de ladite positon de fermeture, et un dispositif de contrôle associé au générateur configuré pour recevoir au moins une information du capteur et permettre l'émission d'un seul flash lumineux lorsque ladite position de fermeture a été détectée par le capteur.

14. Consommable (10, 18) pour le dispositif (11) selon une des revendications 1 à 13, distinct du boîtier du dispositif et permettant un remplacement de la lampe flash du dispositif, **caractérisé en ce qu'**il comprend :
- ladite lampe flash (10) ;
- un caisson (18) pour loger la lampe flash (10), présentant une face d'extrémité avec une fenêtre (19) pour laisser échapper des rayons de la lampe flash (10) ;
- du côté de la face d'extrémité et en périphérie de la fenêtre (19), au moins un évidement dans lequel est placé un ressort qui présente une extrémité mobile entre une position comprimée en retrait permettant à la cavité (C) du dispositif (11) d'être fermée de façon à limiter des échappements de lumière hormis vers l'ouverture (14) et une position avancée pour laquelle la cavité communique avec l'orifice d'aération (O1, 02) du dispositif (11) ;
- au moins un élément de fixation (20), destiné à permettre une fixation amovible entre le caisson du consommable et le boîtier du dispositif (11), présentant une surface de butée qui est orientée suivant un sens opposé par rapport au sens de poussée du ressort.

## Patentansprüche

1. Vorrichtung (11), die zu einer Enthaarung bestimmt ist, umfassend ein Gehäuse, eine Blitzlampe (10), die im Inneren des Gehäuses angeordnet ist, wobei das Gehäuse eine Öffnung (14) aufweist, durch die die elektromagnetische Energie, die von der Blitzlampe (10) stammt, zu der Oberfläche einer Haut übertragen werden kann, wobei das Gehäuse einen Hohlraum (C) aufweist, der an die Blitzlampe (10) angrenzt, **dadurch gekennzeichnet, dass** das Gehäuse Folgendes aufweist:
- mindestens eine Belüftungsöffnung (O1, O2); und
- mindestens ein bewegliches Organ, das zwischen einer ersten Position, der sogenannten Belüftungsposition, in der der Hohlraum (C) mit der Belüftungsöffnung (O1, O2) verbunden ist, und einer zweiten Position, der sogenannten Schließposition, für die der Hohlraum (C) derart geschlossen ist, um das Ausströmen von Licht außerhalb der Öffnung (14) zu begrenzen, beweglich ist.

2. Vorrichtung nach Anspruch 1, wobei das Gehäuse Folgendes aufweist:
- einen ersten Gehäuseabschnitt, der die Öffnung (14) aufweist; und
- einen zweiten Gehäuseabschnitt, der ein translatorisches Verschieben zwischen dem ersten Gehäuseabschnitt und dem zweiten Gehäuseabschnitt ermöglicht, wobei die Blitzlampe (10) mit dem zweiten Gehäuseabschnitt fest verbunden ist, wobei das bewegliche Organ aus einem des ersten und zweiten Abschnitts gebildet ist.

3. Vorrichtung nach Anspruch 2, wobei der erste Gehäuseabschnitt ein Kopf (13) ist und der zweite Gehäuseabschnitt einen Griff (12) bildet, der von einer Hand greifbar ist, wobei das bewegliche Organ ermöglicht, die Blitzlampe (10) näher an das freie Ende des Kopfes (13) zu bringen, wenn der Kopf (13) in Auflage auf einer Fläche ist, die außerhalb der Vorrichtung (11) ist.

4. Vorrichtung nach einem der Ansprüche 1 bis 3, die im Inneren des Gehäuses Folgendes aufweist:
- einen Kuppler (15), der es ermöglicht, die elektromagnetische Energie, die von der Blitzlampe (10) stammt, auf die Oberfläche einer Haut zu lenken;
- einen Filter (17), der zwischen der Blitzlampe (10) und dem Kuppler (15) angeordnet ist;
- eine erste Befestigungsanordnung (22, 25), um den Filter (17) in dem Gehäuse zu befestigen; und
- eine zweite Befestigungsanordnung, um die Blitzlampe (10) in dem Gehäuse zu befestigen;
wobei das bewegliche Organ mindestens eine der ersten und zweiten Befestigungsanordnung verschiebt, wobei die Blitzlampe (10) im Wesentlichen gegenüber von und mit Abstand zu dem Filter (17) für die Belüftungsposition angeordnet ist, wobei die Blitzlampe (10) und der Filter (17) für die Schließposition eng einander angenähert sind.

5. Vorrichtung nach Anspruch 4, wobei die erste Befestigungsanordnung (22, 25) einen translatorischen Freiheitsgrad zwischen dem Kuppler (15) und der Blitzlampe (10) besitzt, wobei die erste Befestigungsanordnung (22, 25) mindestens durch ein elastisches Vorspannorgan (23), das am Umfang des Kupplers (15) angeordnet ist, in Abstand von dem Kuppler (15) gehalten wird.

6. Vorrichtung nach einem der Ansprüche 4 und 5, wobei die erste Befestigungsanordnung (22, 25) einen röhrenförmigen Träger (22) aufweist, der auf der Seite, die dem Kuppler (15) gegenüberliegt, elastische Einrastzungen (25) aufweist, die zwischen sich den Filter (17) halten, wobei das Gehäuse einen röhrenförmigen Abschnitt (26) aufweist, der den Träger (22) umgibt und ermöglicht, den Träger (22) zu führen.

7. Vorrichtung nach einem der Ansprüche 4 bis 6, wobei die zweite Befestigungsanordnung mit der Blitzlampe (10) ein Verbrauchsteil (10, 18) bildet, das aus dem Gehäuse herausnehmbar ist, und einen Kasten (18) aufweist, um die Blitzlampe (10) aufzunehmen, wobei der Kasten (18) Folgendes aufweist:
- eine Stirnfläche, die ein Fenster (19) aufweist, um die Strahlen der Blitzlampe (10) heraustreten zu lassen;
- erste Befestigungselemente (20), die Enden definieren, die der Stirnfläche entgegengesetzt sind;
- zweite Befestigungselemente (21), die am Umfang des Fensters (19) angeordnet sind;
wobei die ersten Befestigungselemente (20) konfiguriert sind, um mit mindestens einem länglichen Organ (30) entlang einer ersten Richtung einzugreifen, während die zweiten Befestigungselemente (21) konfiguriert sind, um mit mindestens einem länglichen Organ (30) entlang einer zweiten Richtung einzugreifen, die senkrecht zu der ersten Richtung ist.

8. Vorrichtung nach Anspruch 7, wobei das Gehäuse im Inneren eine Leitfläche (27) eines Luftstroms parallel zu dem Filter (17) aufweist, ausgehend von der sich entlang einer Richtung, die im Wesentlichen senkrecht zu dem Filter (17) ist, das längliche Organ (30) erstreckt.

9. Vorrichtung nach einem der Ansprüche 7 und 8, wobei das längliche Organ (30) eine Schulter (30b) aufweist und in einem Stangenendabschnitt endet, wobei der Kasten (18) mindestens eine Ausnehmung mit einem Boden und eine Feder aufweist, die in der Ausnehmung vorgesehen ist, wobei die Ausnehmung ermöglicht, mindestens teilweise das längliche Organ (30) aufzunehmen, wobei die Feder ein erstes Ende, das sich auf den Boden aufstützt, und ein zweites Ende aufweist, das sich auf die Schulter (30b) des länglichen Organs (30) aufstützt, wobei die Feder für die Schließposition, in der die Blitzlampe (10) und der Filter (17) eng einander angenähert sind, zusammengedrückt ist.

10. Vorrichtung nach einem der Ansprüche 7 bis 9, wobei die ersten Befestigungselemente (20) metallische, leitende Stromversorgungselemente (33) zum Versorgen der Blitzlampe (10) aufweisen.

11. Vorrichtung nach einem der Ansprüche 9 bis 10, wobei die zweite Befestigungsanordnung einen Kasten (18) aufweist, der es ermöglicht, die Blitzlampe (10) aufzunehmen und metallische, leitende Stromversorgungselemente (33) aufweist, die sich auf der gegenüberliegenden Seite des Kupplers (15) erstrecken, wobei der zweite bewegliche Gehäuseabschnitt mindestens ein im Wesentlichen zylindrisches Metallteil (32) aufweist, auf dem die leitenden Stromversorgungselemente (33) befestigt werden.

12. Vorrichtung nach Anspruch 11, wobei der Kasten (18) ein Fenster (19) aufweist, das mehrere Strahlen der Blitzlampe (10) entlang einer bestimmten allgemeinen Ausrichtung heraustreten lässt, wobei der erste Gehäuseabschnitt mindestens ein Führungsteil des Kastens (18) aufweist, das entlang einer Achse langgestreckt ist, die parallel zu der bestimmten allgemeinen Ausrichtung ist.

13. Vorrichtung nach einem der Ansprüche 1 bis 12, umfassend einen Generator, an den die Blitzlampe (10) angeschlossen ist, einen Sensor, der geeignet ist, eine Information auszugeben, die für die Belegung der Schließposition durch das bewegliche Organ repräsentativ ist, und eine Kontrollvorrichtung, die an den Generator angeschlossen ist, die konfiguriert ist, um mindestens eine Information des Sensors zu empfangen und das Emittieren eines einzigen Lichtblitzes zu ermöglichen, wenn die Schließposition von dem Sensor erfasst worden ist.

14. Verbrauchsteil (10, 18) für die Vorrichtung (11) nach einem der Ansprüche 1 bis 13, das von dem Gehäuse der Vorrichtung verschieden ist und ein Ersetzen der Blitzlampe der Vorrichtung ermöglicht, **dadurch gekennzeichnet, dass** es Folgendes aufweist:
- die Blitzlampe (10);
- einen Kasten (18), um die Blitzlampe (10) aufzunehmen, der eine Stirnfläche mit einem Fenster (19) aufweist, um Strahlen der Blitzlampe (10) heraustreten zu lassen;
- auf der Seite der Stirnfläche und am Umfang des Fensters (19) mindestens eine Ausnehmung, in der eine Feder angeordnet ist, die ein Ende aufweist, das zwischen einer zusammengedrückten zurückgezogenen Position, die dem Hohlraum (C) der Vorrichtung (11) ermöglicht, derart geschlossen zu werden, um das Ausströmen von Licht außer zu der Öffnung (14) zu begrenzen, und einer vorgeschobenen Position, für die der Hohlraum mit der Belüftungsöffnung (O1, O2) der Vorrichtung (11) verbunden ist, beweglich ist;
- mindestens ein Befestigungselement (20), das dazu bestimmt ist, eine bewegliche Befestigung zwischen dem Kasten des Verbrauchsteils und dem Gehäuse der Vorrichtung (11) zu ermöglichen, das eine Anschlagfläche aufweist, die in einer Richtung ausgerichtet ist, die entgegengesetzt zur Schubrichtung der Feder ist.

## Claims

1. Device (11) intended for hair removal, comprising a housing, a flashlamp (10) arranged inside the housing, the housing having an opening (14) through which electromagnetic energy originating from the flashlamp (10) can be transmitted towards a skin surface, the housing comprising a cavity (C) adjacent to the flashlamp (10), **characterized in that** the housing comprises:
- at least one aeration orifice (01,02); and
- at least one mobile component, which can be moved between a first so-called aeration position, in which said cavity (C) communicates with said aeration orifice (01,02) and a second so-called closed position, for which said cavity (C) is closed in order to limit the light escaping from the opening (14).

2. Device according to claim 1, in which the housing has:
- a first housing portion that comprises said opening (14); and
- a second housing portion allowing a displacement in translation between the first housing portion and the second housing portion, the flashlamp (10) being integral with the second housing portion, the mobile component being formed by one of said first and second portions.

3. Device according to claim 2, in which the first housing portion is a head (13) and the second housing portion forms a handle (12) which can be grasped by a hand, said mobile component making it possible to move the flashlamp (10) towards the free end of the head (13) when the head (13) is supported on a surface outside the device (11).

4. Device according to one of claims 1 to 3, comprising inside the housing:
- a coupler (15) making it possible to direct the electromagnetic energy originating from the flashlamp (10) onto a skin surface;
- a filter (17) inserted between the flashlamp (10) and the coupler (15);
- a first fixing arrangement (22, 25) for fixing the filter (17) in the housing; and
- a second fixing arrangement for fixing the flashlamp (10) in the housing;
said mobile component displacing at least one of the first and second fixing arrangements, the flashlamp (10) being arranged substantially opposite and at a distance from the filter (17) for said aeration position, the flashlamp (10) and the filter (17) being moved close together for said closed position.

5. Device according to claim 4, in which the first fixing arrangement (22, 25) has a degree of freedom in translation between the coupler (15) and the flashlamp (10), the first fixing arrangement (22, 25) being held at a distance from the coupler (15) by at least one elastic stress component (23) placed on the periphery of the coupler (15).

6. Device according to one of claims 4 and 5, in which the first fixing arrangement (22, 25) comprises a tubular support (22) that comprises on the side opposite the coupler (15), elastic clipping tabs (25) retaining said filter (17) between them, the housing comprising a tubular portion (26) surrounding the support (22) and making it possible to guide the support (22).

7. Device according to one of claims 4 to 6, in which the second fixing arrangement forms with the flashlamp (10) an expendable unit (10, 18) that can be removed from the housing and comprises a box (18) for accommodating the flashlamp (10), said box (18) having:
- an end surface having a window (19) to allow the rays from the flashlamp (10) to escape;
- first fixing elements (20) defining ends opposite said end surface;
- second fixing elements (21) arranged on the periphery of the window (19);
the first fixing elements (20) being configured to engage with at least one component (30) elongated in a first direction, whilst the second fixing elements (21) are configured to engage with at least one component (30) elongated in a second direction perpendicular to the first direction.

8. Device according to claim 7, in which the inside of the housing has a surface (27) for guiding a flow of air parallel to the filter (17) and from which said elongated component (30) extends in a general direction perpendicular to the filter (17).

9. Device according to one of claims 7 and 8, in which said elongated component (30) has a shoulder (30b) and ends in an end rod portion, the box (18) comprising at least one recess with a base and a spring provided in the recess, the recess making it possible to receive at least part of said elongated component (30), the spring having a first end supported on the base and a second end supported on the shoulder (30b) of the elongated component (30), said spring being compressed for said closed position in which the flashlamp (10) and the filter (17) are moved close together.

10. Device according to one of claims 7 to 9, in which the first fixing elements (20) comprise metal power conducting elements (33) to power the flashlamp (10).

11. Device according to one of claims 9 to 10, in which the second fixing arrangement comprises a box (18) making it possible to accommodate the flashlamp (10) and having metal power conducting elements (33) extending the opposite way to the coupler (15), the second mobile housing portion having at least one substantially cylindrical metal piece (32) to which the power conducting elements (33) are fixed.

12. Device according to claim 11, in which the box (18) has a window (19) that allows a plurality of rays from the flashlamp (10) to escape in a determined general orientation, the first housing portion having at least one elongated guiding part of the box (18) along an axis parallel to said determined general orientation.

13. Device according to one of claims 1 to 12, comprising a generator to which are connected the flashlamp (10), a sensor capable of delivering a piece of information representative of the occupation by said mobile component of said closed position, and a control device associated with the generator configured to receive at least one piece of information from the sensor and allow the emission of a single flash of light when said closed position has been detected by the sensor.

14. Expendable unit (10, 18) for the device (11) according to one of claims 1 to 13, distinct from the device housing and suitable for replacement of the flashlamp of the device, **characterized in that** it comprises:
- said flashlamp (10);
- a box (18) for accommodating the flashlamp (10), having an end surface with a window (19) to allow the rays from the flashlamp (10) to escape;
- on the side of the end surface and on the periphery of the window (19), at least one recess in which a spring is placed, which has an end mobile between a compressed retracted position allowing the cavity (C) of the device (11) to be closed in order to limit light escaping except towards the opening (14) and an extended position for which the cavity communicates with the aeration orifice (owl, 02) of the device (11);
- at least one element (20) for fixing the box of the expendable unit to the housing of the device (11) in removable manner, having a stop surface that is oriented in an opposite direction in relation to the thrust direction of the spring.
